# EUROPÄISCHE PATENTSCHRIFT

(11) **EP 0 450 564 B1**
(45) Veröffentlichungstag und Bekanntmachung des Hinweises auf die Patenterteilung: **06.08.1997**
(21) Anmeldenummer: 91105176.1
(22) Anmeldetag: 02.04.1991
(51) Int. Cl.: D02J 1/18, G01N 33/36

(54) **Verfahren und Vorrichtung zum Führen eines Faserkabels**
Method and apparatus for grinding a yarn tow
Procédé et dispositif pour guider un câble de filaments

(30) Priorität: 04.04.1990 DE 4010831
(43) Veröffentlichungstag der Anmeldung: 09.10.1991
(73) Patentinhaber: HOECHST AKTIENGESELLSCHAFT, 65926 Frankfurt am Main (DE)
(72) Erfinder: Neuert, Richard, W-8420 Kelheim (DE); Huber, Bernd, W-8420 Kelheim (DE)

(56) Entgegenhaltungen:
- EP-A- 0 271 728
- DD-A- 214 159
- US-A- 3 503 100
- US-A- 3 874 030

## Beschreibung

Die Erfindung betrifft ein Verfahren und eine Vorrichtung zum Führen eines endlosen laufenden Faserkabels bei der Synthesefaserherstellung. Bei einem derartigen Faserkabel handelt es sich um eine Filamentschar aus einigen tausend bis einigen Millionen Filamenten, insbesondere vor dem Eintritt in eine Stauchkammerkräuselmaschine.

Es ist bekannt, daß die Qualität, insbesondere die Gleichmäßigkeit gekräuselter Synthesefaserkabel oder daraus hergestellter Spinnfasern, wesentlich von der Gleichmäßigkeit des einlaufenden Faserkabels beeinflußt wird. Es sind deshalb auch schon viele Vorrichtungen beschrieben worden, die zu einem Kabel gleichmäßiger Dicke führen sollen, z.B. ballige Walzen (DE 17 85 028), Fadenführer mit Nuten (US 43 01 579), seitliche Fadenführungen (DE 14 35 438) oder doppelkegelstumpfförmige Rollen (DE 16 60 291).
Vielfach wurden auch Verfahren beschrieben, bei denen das einlaufende Kabel aus einer Vielzahl von Teilkabeln zusammengesetzt wird (GB 962 516, DE 21 44 763, EP 199 239). Diese Verfahren gehen im Prinzip davon aus, daß sich die Fehler der Teilkabel durch das Fachen im Mittelwert aufheben.
Aus der DE 33 06 867 sind ein Verfahren und eine Vorrichtung zum Führen eines endlosen laufenden Faserkabels vor dem Eintritt in eine Stauchkammerkräuselmaschine bekannt, bei denen die Spannung des Faserkabels laufend gemessen und in Abhängigkeit hiervon
die Geschwindigkeit des Lieferwerks der Kräuselkammermaschine gesteuert wird. Hierdurch läßt sich jedoch nicht die Geometrie des Faserkabels optimieren.

Die DD-A-2 141 59 beschreibt ein Verfahren, worin das Beugungsbild eines bereits texturierten, also gekräuselten einzelnen Fadens herangezogen wird, um das Ergebnis der Kräuselung zu beurteilen. Die Höhe, Breite oder das Querschnittsprofil eines Faserkabels wird nicht bestimmt.

Der Erfindung liegt die Aufgabe zugrunde, ein Verfahren und eine Vorrichtung zum Führen eines endlosen laufenden Faserkabels bei der Synthesefaserherstellung anzugeben, die eine Optimierung der Geometrie des Faserkabels erlauben.

Zur Lösung dieser Aufgabe ist das erfindungsgemäße Verfahren dadurch gekennzeichnet, daß mindestens eine geometrische Größe oder eine für diese geometrische Größe repräsentative Eigenschaft des Faserkabels berührungslos abgefühlt und die geometrische Größe des Faserkabels so gesteuert wird, daß sie innerhalb eines vorgegebenen Sollwertbereiches bleibt.

Das berührungslose Abfühlen erfolgt zweckmäßigerweise auf elektrooptischem Weg. Vorzugsweise wird dabei die Intensität eines vom Faserkabel durchgelassenen oder reflektierten Lichtstrahles gemessen. Dies erlaubt eine Aussage über das Kabelprofil (Dicke und Breite des Faserkabels) sowie die Lage des Faserkabels in Querrichtung.

Als charakteristische Größen für das Kabelprofil wird vorzugsweise die Dicke des Faserkabels über seiner Breite und/oder die Breite des Faserkabels und/oder die Lage des Faserkabels in Querrichtung abgefühlt.

Diese geometrischen Größen werden dann - vorzugsweise durch am Faserkabel angreifende Umlenkmittel-kontinuierlich so gesteuert, daß sie innerhalb vorgegebener Sollwertbereiche bleiben.

Das Abfühlen und Steuern wird vorteilhafterweise abschnittsweise in über der Breite des Faserkabels verteilten streifenförmigen Teilbereichen durchgeführt.

Auf diese Weise läßt sich zum Beispiel ein optimaler rechteckiger Kabelquerschnitt wie auch eine optimale Lage des Faserkabels in Querrichtung (auf der Transportgalette) aufrechterhaften. Dies erlaubt es, der nachgeschalteten Kräuselmaschine ständig ein Faserkabel optimaler Geometrie vorzulegen. Es wird daher eine gleichmäßige Kräuselung über der gesamten Kabelbreite erreicht. Die Kräuselkammerbreite läßt sich sehr gut nutzen. Außerdem wird die textile Weiterverarbeitkeit der Kabelfaser verbessert.
Es versteht sich, daß das erfindungsgemäße Verfahren nicht nur vor Eintritt in eine Kräuselmaschine, sondern ganz allgemein dort einsetzbar ist, wo es auf eine optimale Kabelgeometrie ankommt, z.B. beim Einlauf in den Trockner.

Eine Vorrichtung zum Durchführen des erfindungsgemäßen Verfahrens ist gekennzeichnet durch eine berührungslos arbeitende Sensoreinrichtung(10;12), die die geometrische Größe bzw. die hierfür repräsentative Eigenschaft des Faserkabels (2) mißt und ein entsprechendes elektrisches Meßsignal erzeugt, eine Signalverarbeitungseinrichtung ein (14), die in Abhängigkeit von dem Meßsignal Steuersignal erzeugt, und eine Steuereinheit (16), die in Abhängigkeit von dem Steuersignal die geometrische Größe des Faserkabels (2) steuert.

Vorzugsweise ist vorgesehen, daß die Sensoreinrichtung eine das Faserkabel beleuchtende Lichtquelle (10) und eine elektronische Kamera (12) aufweist, die das vom Faserkabel (2) durchgelassene oder reflektierte Licht erfaßt. Die Kamera kann eine Videokamera sein, ist jedoch vorzugsweise eine CCD-Zeilen- oder Matrixkamera. Die Lichtquelle und die Kamera können auf der gleichen Seite des Faserkabels angeordnet sein. Vorzugsweise sind die Lichtquelle und die Kamera auf voneinander abgewandten Seiten des Faserkabels angeordnet, da das Durchlichtverfahren eine genauere Aussage über die zu überwachenden geometrischen Größen des Faserkabels erlaubt.

Die Steuereinheit (16) weist vorzugsweise eine oder mehrere am Faserkabel angreifende Umlenkeinrichtungen (18;20;22) zum Steuern der Dicke und/oder Breite und/oder Lage des Faserkabels (2) auf. Eine Umlenkeinrichtung zum Steuern der Dicke des Faserkabels weist eine Reihe von mindestens zwei nebeneinander liegenden, senkrecht zur Faserkabelebene und -laufrichtung bewegbaren Umlenkelementen (24) auf, die zum örtlichen Aufweiten des Faserkabels (2) unabhängig voneinander gegen das Faserkabel bewegbar sind. Zum Steuern der Breite und Lage des Faserkabels werden vorzugsweise gerade bzw. gekrümmte Umlenkstangen bzw. -rollen verwendet, die quer zum Faserkabel verlaufen und kippbar bzw. um ihre Längsachse drehbar sind.
Anhand der Zeichnungen werden das erfindungsgemäße Verfahren sowie eine Vorrichtung zum Durchführen dieses Verfahrens näher beschrieben. Es zeigt:
Figur 1 eine Seitenansicht einer Vorrichtung zum Führen eines Faserkabels in schematischer Weise;
Figur 2 eine Draufsicht auf die Vorrichtung nach Figur 1;
Figur 3 einen Bildschirm zur Veranschaulichung eines dem Kabelprofil entsprechenden Meßsignals;
Figur 4 ein Kabelprofil mit einer Umlenkeinrichtung zum Steuern der Dicke des Faserkabels;
Figuren 5, 6 eine Umlenkeinrichtung zum Steuern der Breite des Faserkabels in unterschiedlichen Betriebszuständen;
Figur 7 eine Umlenkeinrichtung zum Steuern der seitlichen Lage des Faserkabels.

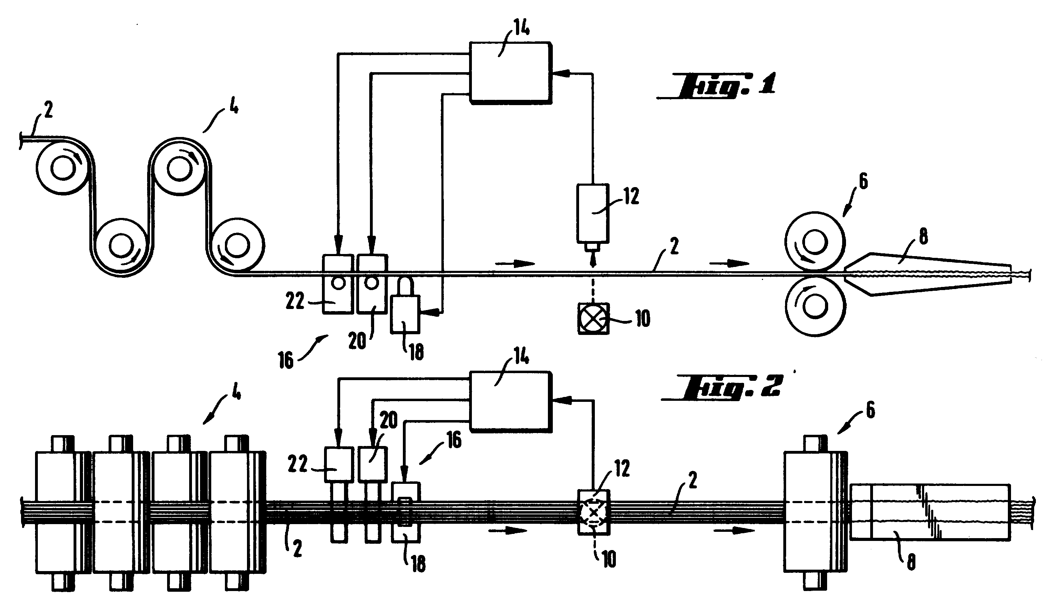

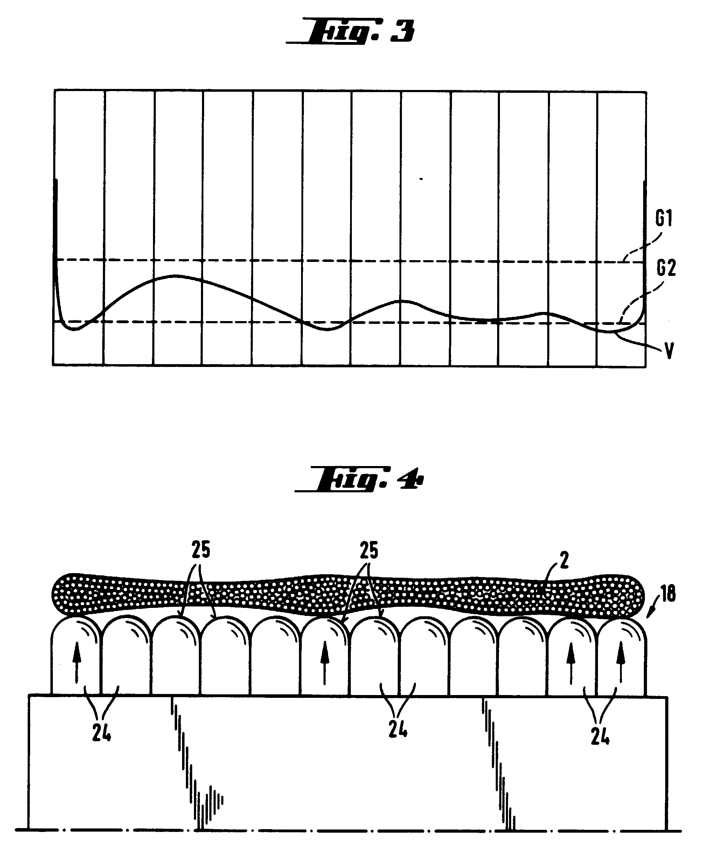

Die in den Figuren 1 und 2 gezeigte Vorrichtung zum Führen eines Faserkabels (2) besitzt ein Einlauflieferwerk (4) und ein Ablauflieferwerk (6), das im dargestellten Ausführungsbeispiel die Einzugswalze einer Kräuselkammer (8) ist. Das Faserkabel (2) besteht aus einer Vielzahl von Filamenten und hat beispielsweise den in Figur 4 gezeigten Querschnitt.

Unterhalb der Laufbahn des Faserkabels (2) ist eine Lichtquelle in Form einer Lampe (10) angeordnet, die einen Lichtstrahl nach oben gegen das Faserkabel (2) richtet. Auf der von der Lichtquelle (10) abgewandten Seite des Faserkabels (2) ist eine Kamera (12) angeordnet, die die Lichtintensität des durch das Faserkabel (2) durchscheinenden Lichtes mißt. Die Kamera (12) ist beispielsweise eine CCD-Zeilenkamera (wie sie z.B. von der Firma Honeywell unter der Bezeichnung HVS 256 vertrieben wird). Die Zeilenkamera sollte eine Auflösung von mindestens 128 Grauwertstufen haben und erfaßt im konkreten Ausführungsbeispiel 256 Grauwertstufen.

Das von der Kamera (12) erzeugte Meßsignal, das die Lichtintensität des durch das Faserkabel (2) durchscheinenden Lichtes über der Breite des Faserkabels darstellt, wird einer Signalverarbeitungseinrichtung (14) in Form eines Rechners zugeführt, die Steuersignale zur Betätigung einer Steuereinheit 16 erzeugt.

Die Steuereinheit (16) besteht im dargestellten Ausführungsbeispiel aus einer Umlenkeinrichtung (18) zum Steuern der Dicke des Faserkabels (2), einer Umlenkeinrichtung (20) zum Steuern der Breite des Faserkabels (2) und einer Umlenkeinrichtung (22) zum Steuern der Lage des Faserkabels (2) in Querrichtung. Die Umlenkeinrichtungen (18, 20 und 22) sind hintereinander so angeordnet, daß - in Kabellaufrichtung gesehen - zunächst die seitliche Lage, dann die Breite und dann die Dicke des Faserkabels 2 gesteuert werden.

Wie in Figur 4 schematisch angedeutet ist, besteht die Umlenkeinrichtung (18) aus einer Reihe von nebeneinanderliegenden Umlenkelementen (24) in Form von vertikal angeordneten Stäben, die unabhängig voneinander axial verschiebbar sind. Jedes Umlenkelement (24) ist mit einem abgerundeten Ende (25) versehen, das in das Faserkabel (2) eindringen kann, um das Faserkabel (2) örtlich aufzuweiten und hierdurch seine Dicke zu verringern.

Wie in den Figuren 5 und 6 schematisch angedeutet ist, besteht die Umlenkeinrichtung (20) aus einer gekrümmten Umlenkstange (26), die quer zur Längsrichtung des Faserkabels (2) verläuft. Die Umlenkstange (26) ist um ihre Längsachse drehbar, so daß das Faserkabel (2) an der konvexen oder konkaven Seite der Umlenkstange (26) anliegt. Liegt das Faserkabel (2) an der konkaven Seite der Umlenkstange (26) an (Figur 5), so hat dies eine Verringerung der Breite des Faserkabels (2) zur Folge. Greift dagegen die Umlenkstange (26) mit ihrer konvexen Seite am Faserkabel (2) an, so vergrößert dies die Breite des Faserkabels (2).

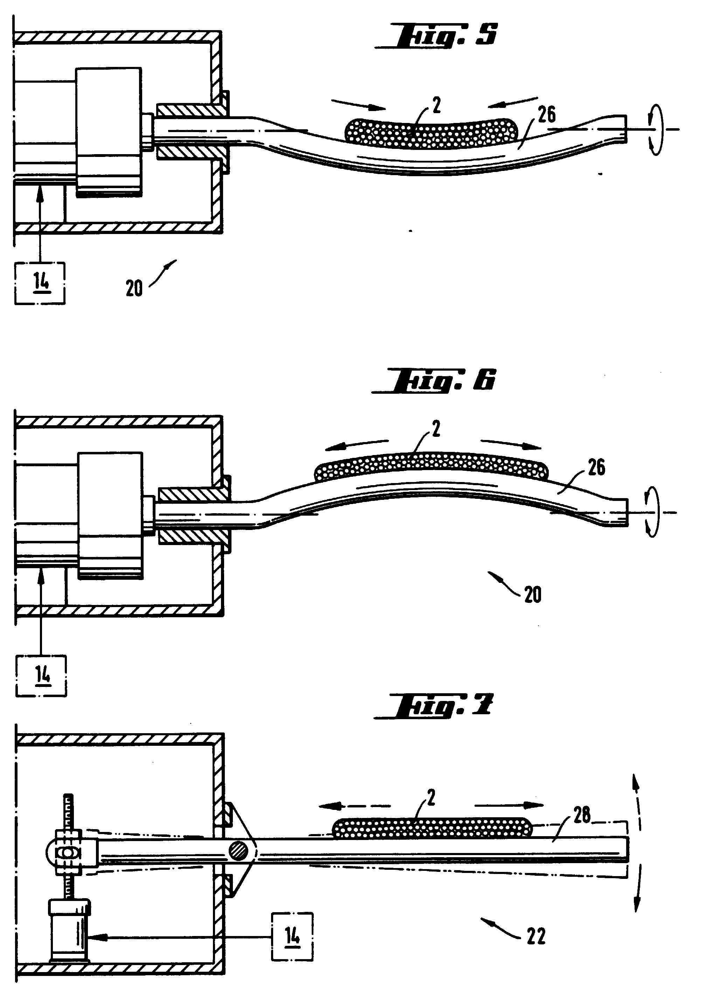

Die in Figur 7 gezeigte Umlenkeinrichtung (22) zum Steuern der Lage des Faserkabels (2) besteht aus einer geradlinigen Umlenkstange bzw. -rolle (28), die quer zur Längsrichtung des Faserkabels (2) verläuft. Wie in Figur 7 schematisch angedeutet ist, ist die Umlenkstange (28) an ihrem (in der Figur) linksseitigen Ende kippbar gelagert. Bei einer Schwenkbewegung der Umlenkstange (28) um mindestens ± 0,1° ergibt sich eine seitliche Verschiebung des Faserkabels (2) nach rechts oder links (in Figur 7).

Es wird nun die Betriebsweise der beschriebenen Vorrichtung erläutert. Es sei angenommen, daß das Faserkabel (2) zu einem bestimmten Zeitpunkt das in Figur 4 gezeigte Profil besitzt.

Die Zeilenkamera (12) erzeugt dann in Abhängigkeit von dem durch das Faserkabel (2) durchscheinenden Licht ein analoges Meßsignal V, das die Dickenverteilung des Faserkabels (2) in der Meßebene darstellt. Dieses analoge Meßsignal V wird im Rechner (14) weiterverarbeitet und kann am Bildschirm des Rechners (14) dargestellt werden, wie in Figur 3 schematisch angedeutet ist. In Figur 3 ist ebenfalls ein Sollwertbereich für das Signal V mit einer oberen und unteren Grenze G1 bzw. G2 eingezeichnet.

Wie in Figur 3 ferner gezeigt ist, ist der Bildschirm in ein Raster mit einer Anzahl (n) von streifenförmigen Teilbereichen unterteilt, wobei im dargestellten Ausführungsbeispiel n = 12 ist. Jedem streifenförmigen Teilbereich ist ein Umlenkelement (24) der Umlenkeinrichtung 18 (Figur 4) zugeordnet. Verletzt das die Dickenverteilung darstellende Signal V in einem oder mehreren der rasterförmigen Teilbereiche die obere oder untere Grenze G1 bzw. G2, so gibt der Rechner (14) entsprechende Steuersignale an die Umlenkeinrichtung (18) ab, um die zugehörigen Umlenkelemente (24) zu verschieben.

Im dargestellten Ausführungsbeispiel verletzt das Signal V die untere Grenze G2 im ersten, sechsten, elften und zwölften Teilbereich (gesehen von links). Dies bedeutet, daß das Faserkabel (2) in diesen Teilbereichen eine zu große Dicke hat. Somit werden das erste, sechste, elfte und zwölfte Umlenkelement (24) in das Faserkabel (2) hineinverschoben, um das Faserkabel in diesen Teilbereichen aufzuweiten und somit seine Dicke örtlich zu verringern.

Der Vergleich des Signals V mit dem durch die Grenzen G1, G2 festgelegten Sollwertbereich kann zusätzlich an einer Diodenleiste (nicht gezeigt) dargestellt werden. Beispielsweise können jedem streifenförmigen Teilbereich zwei Dioden zugeordnet werden, wobei die eine Diode bei Verletzung der oberen Grenze und die andere Diode bei Verletzung der unteren Grenze aufleuchtet und die beiden Dioden kein Signal zeigen, wenn sich das Signal V innerhalb des Sollwertbereiches befindet.

Das Signal V wird auch zur Bestimmung der Ist-Breite und Ist-Lage des Faserkabels 2 herangezogen. Bei entsprechenden Grenzwertverletzungen gibt der Rechner (14) entsprechende Steuersignale an die Umlenkeinrichtungen (20) und (22) zum Korrigieren der Breite und Lage des Faserkabels ab.
Auf diese Weise werden das Kabelprofil (Dicke und Breite) sowie die Lage des Faserkabels im on-line Verfahren kontinuierlich in optimalen Bereichen gehalten.

Treten bei der im Rechner (14) vorgenommenen Auswertung des Signals V Grenzwertverletzungen auf, so kann ein Fehlerprotokoll ausgegeben werden, in dem der Zeitpunkt sowie die Klassifizierung der Fehler angegeben sind. Die CCD-Zeilenkamera sollte beispielsweise im Zeitabstand von mindestens 5 ms eine Aufnahme machen.

Statt einer CCD-Zeilenkamera kann natürlich auch eine CCD-Matrixkamera eingesetzt werden. In diesem Fall ergibt sich statt einer einzelnen Meßzeile ein matrixförmiges Meßfeld, das eine entsprechende Signalauswertung im Rechner verlangt. Das Verfahren zur Steuerung der Kabelgeometrie ist jedoch das gleiche wie bei Verwendung einer Zeilenkamera.

## Patentansprüche

1. Verfahren zum Führen eines endlosen laufenden Faserkabels bei der Synthesefaserherstellung, bei dem eine Eigenschaft des Faserkabels berührungslos abgefühlt, laufend überwacht und in Abhängigkeit von dieser Überwachung diese Eigenschaft des Faserkabels gesteuert wird, dadurch gekennzeichnet, daß die Dicke des Faserkabels über seine Breite und/oder die Breite des Faserkabels und/oder die Lage des Faserkabels in Querrichtung abgefühlt wird und mindestens eine dieser Eigenschaften des Faserkabels so gesteuert wird, daß sie innerhalb eines vorgegebenenen Sollwertbereiches bleibt.

2. Verfahren nach Anspruch 1, dadurch gekennzeichnet, daß das Abfühlen auf elektrooptischem Weg erfolgt.

3. Verfahren nach Anspruch 1 oder 2, dadurch gekennzeichnet, daß die Lichtintensität eines vom Faserkabel durchgelassenen oder reflektierten Lichtstrahles abgefühlt wird.

4. Verfahren nach Anspruch 3, dadurch gekennzeichnet, daß das Steuem durch am Faserkabel angreifende Umlenkmittel erfolgt.

5. Verfahren nach einem der Ansprüche 1-4, dadurch gekennzeichnet, daß das Abfühlen und Steuern abschnittsweise in über der Breite des Faserkabels verteilten streifenförmigen Teilbereichen durchgeführt wird.

6. Vorrichtung zum Durchführen des Verfahrens nach einem der vorhergehenden Ansprüche mit einer berührungslos arbeitenden Sensoreinrichtung, dadurch gekennzeichnet, daß die Sensoreinrichtung (10, 12) die Dicke des Faserkabels (2) über seine Breite und/oder die Breite des Faserkabels und/oder die Lage des Faserkabels in Querrichtung mißt und ein entsprechendes elektrisches Meßsignal erzeugt, eine Signalverarbeitungseinrichtung (14) in Abhängigkeit von dem Meßsignal ein Steuersignal erzeugt, und eine Steuereinheit (16) in Abhängigkeit von dem Steuersignal die gemessenen geometrischen Größen des Faserkabels (2) steuert.

7. Vorrichtung nach Anspruch 6, dadurch gekennzeichnet, daß die Sensoreinrichtung eine das Faserkabel beleuchtende Lichtquelle (10) und eine elektronische Kamera (12) aufweist, die das vom Faserkabel (2) durchgelassene oder reflektierte Licht erfaßt.

8. Vorrichtung nach Anspruch 7, dadurch gekennzeichnet, daß die Lichtquelle (10) und die Kamera (12) auf voneinander abgewandten Seiten des Faserkabels angeordnet sind.

9. Vorrichtung nach Anspruch 7 oder 8, dadurch gekennzeichnet, daß die Kamera (12) eine Zeilen- oder Matrixkamera ist, die einen Charge Coupled Device (CCD)-Bildsensor enthält.

10. Vorrichtung nach Anspruch 9, dadurch gekennzeichnet, daß die Kamera (12) zur Erfassung von mindestens 128 Grauwertstufen ausgebildet ist.

11. Vorrichtung nach einem der Ansprüche 6 bis 10, dadurch gekennzeichnet, daß die Steuereinheit (16) mindestens eine am Faserkabel (2) angreifende Umlenkeinrichtung (18;20;22) zum Steuern der Dicke und/oder Breite und/oder Lage des Faserkabels (2) aufweist.

12. Vorrichtung nach Anspruch 11, dadurch gekennzeichnet, daß die Umlenkeinrichtung (18) zum Steuern der Dicke des Faserkabels (2) eine Reihe von mindestens zwei nebeneinander liegenden, senkrecht zur Faserkabelebene und -laufrichtung bewegbaren Umlenkelementen (24) aufweist, die zum örtlichen Aufweiten des Faserkabels (2) unabhängig voneinander gegen das Faserkabel bewegbar sind.

13. Vorrichtung nach Anspruch 12, dadurch gekennzeichnet, daß die Umlenkelemente aus axial verschiebbaren Stäben (24) bestehen, die mit abgerundeten Enden (25) an dem Faserkabel (2) angreifen.

14. Vorrichtung nach einem der Ansprüche 11 bis 13, dadurch gekennzeichnet, daß die Umlenkeinrichtung (20) zum Steuern der Breite des Faserkabels (2) mindestens eine gekrümmte Umlenkstange (26) aufweist, deren Längsachse senkrecht zur Kabellaufrichtung und im wesentlichen parallel zur Kabelebene verläuft und die um ihre Längsachse drehbar ist.

15. Vorrichtung nach einem der Ansprüche 11 bis 14, dadurch gekennzeichnet, daß die Umlenkeinrichtung (22) zum Steuern der Lage des Faserkabels (2) in Querrichtung mindestens eine gerade Umlenkstange bzw. -rolle (28) aufweist, deren Längsachse senkrecht zur Kabellaufrichtung und parallel zur Kabelebene verläuft und die um eine parallel zur Kabellaufrichtung verlaufende Achse kippbar ist.

## Claims

1. A process for guiding a continuous moving tow in synthetic fiber manufacture in which a property of the tow is subjected to contactless sensing, continuously monitored and controlled as a function of this monitoring, characterized in that it comprises sensing the thickness of the tow across its width and/or the width of the tow and/or the position of the tow in the transverse direction and controlling at least one of these properties of the tow in such a way that it stays within a predetermined target value range.

2. The process of claim 1, characterized in that the sensing is effected electro-optically.

3. The process of claim 1 or 2, characterized in that the intensity of a light beam transmitted or reflected by the tow is sensed.

4. The process of claim 3, characterized in that the controlling is effected by tow-engaging deflecting means.

5. The process of any one of the preceding claims 1-4, characterized in that the sensing and controlling is carried out section by section in strip-shaped subsidiary regions distributed across the width of the tow.

6. Apparatus for carrying out the process of any one of the preceding claims, comprising a non-contact sensor means, characterized in that the sensor means (10, 12) measures the thickness of the tow (2) across its width and/or the width of the tow and/or the position of the tow in the transverse direction and generates a corresponding electrical measurement signal, a signal processing means (14) generates a control signal as a function of the measurement signal, and a control unit (16) controls the measured geometric variables of the tow (2) as a function of the control signal.

7. Apparatus of claim 6, characterized in that the sensor means comprises a light source (10) which illuminates the tow and an electronic camera (12) which detects the light transmitted or reflected by the tow (2).

8. Apparatus of claim 7, characterized in that the light source (10) and the camera (12) are arranged on opposite sides of the tow.

9. Apparatus of claim 7 or 8, characterized in that the camera (12) is a line or matrix camera which comprises a charge coupled device (CCD) image sensor.

10. Apparatus of claim 9, characterized in that the camera (12) is capable of detecting at least 128 gray tone levels.

11. Apparatus of any one of claims 6 to 10, characterized in that the control unit (16) comprises at least one tow (2)-engaging deflecting means (18; 20; 22) for controlling the thickness and/or width and/or position of the tow (2).

12. Apparatus of claim 11, characterized in that the deflecting means (18) for controlling the thickness of the tow (2) comprises a series of at least two side-by-side deflecting elements (24) which are movable perpendicularly to the tow plane and transport direction and which are movable independently of each other in the direction of the tow (2) for the purpose of widening it out locally.

13. Apparatus of claim 12, characterized in that the deflecting elements comprise axially displaceable rods (24) which engage with the tow (2) with rounded-off ends (25).

14. Apparatus of any one of claims 11 to 13, characterized in that the deflecting means (20) for controlling the width of the tow (2) comprises at least one curved deflecting bar (26) whose longitudinal axis extends perpendicularly to the tow transport direction and essentially parallel to the tow plane and which is rotatable about its longitudinal axis.

15. Apparatus of any one of claims 11 to 14, characterized in that the deflecting means (22) for controlling the position of the tow (2) in the transverse direction comprises at least one straight deflecting bar or roller (28) whose longitudinal axis extends perpendicularly to the tow transport direction and parallel to the tow plane and which is tiltable about an axis which extends parallel to the tow transport direction.

## Revendications

1. Procédé pour conduire un câble de fibres sans fin qui se déplace dans le processus de préparation de fibres synthétiques, dans lequel on surveille constamment sans contact, l'une des caractéristiques du câble de fibres, et en fonction de cette surveillance, on contrôle cette caractéristique du câble de fibres, procédé caractérisé en ce que l'on détecte dans le sens transversal la grosseur du câble de fibres par sa largeur et/ou la largeur du câble de fibres et/ou la position du câble de fibres, et au moins l'une de ces caractéristiques du câble de fibres est contrôlée de façon à se maintenir à l'intérieur d'un intervalle de valeurs requises établies préalablement.

2. Procédé selon la revendication 1, caractérisé en ce que la détection est effectuée par des méthodes électrooptiques.

3. Procédé selon la revendication 1, caractérisé en ce que l'on détecte l'intensité de la lumière d'un faisceau lumineux transmise ou réfléchie par le câble de fibres.

4. Procédé selon la revendication 3, caractérisé en ce que l'on le câble de fibres à l'aide d'un dispositif déflecteur agissant sur le câble de fibres.

5. Procédé selon l'une des revendications précédentes 1 à 4, caractérisé en ce qu'on effectue la détection et le contrôle par étapes sur des sous-sections sous forme de bandes réparties sur la largeur du câble de fibres.

6. Dispositif pour la mise en oeuvre du procédé selon l'une des revendications précédentes à l'aide d'un dispositif de capteurs qui opère sans contact, caractérisé en ce que le dispositif de capteurs (10, 12) mesure dans le sens transversal la grosseur du câble de fibres (2) par sa largeur et/ou la largeur du câble de fibres et/ou la position du câble de fibres, et engendre un signal de mesure électrique correspondant, un dispositif de traitement de signal (14), en fonction du signal de mesure, génère un signal de contrôle, et une unité de contrôle (16) contrôle, en fonction du signal de mesure, les grandeurs géométriques mesurées sur le câble de fibres (2).

7. Dispositif selon la revendication 6, caractérisé en ce que le dispositif de capteurs présente une source lumineuse (10) illuminant le câble de fibres et une caméra électronique (12), qui capte la lumière transmise ou réfléchie par le câble de fibres (2).

8. Dispositif selon la revendication 7, caractérisé en ce que la source lumineuse (10) et la caméra (12) sont disposées sur les côtés opposées du câble de fibres.

9. Dispositif selon la revendication 7 ou 8, caractérisé en ce que la caméra (12) est une caméra à lignes vidéo ou à matrice, qui comporte comme détecteur d'image un capteur à transfert de charge.

10. Dispositif selon la revendication 9, caractérisé en ce que la caméra (12) est constituée de façon à capter au moins 128 degré de valeur de gris.

11. Dispositif selon l'une des revendications 6 à 10, caractérisé en ce que l'unité de contrôle (16) présente au moins un dispositif déflecteur (18 ; 20 ; 22) agissant sur le câble de fibres (2) pour contrôler la grosseur et/ou la largeur et/ou la position du câble de fibres (2).

12. Dispositif selon la revendication 11, caractérisé en ce que le dispositif déflecteur (18) pour le contrôle de la grosseur du câble de fibres (2) présente une série d'au moins deux éléments déflecteurs mobiles (24), disposés côte à côte, perpendiculairement au niveau du câble de fibres et au sens du déplacement du câble de fibres, lesdits éléments déflecteurs, en vue d'un élargissement local du câble de fibres (2), sont mobiles indépendamment l'un de l'autre contre le câble de fibres.

13. Dispositif selon la revendication 12, caractérisé en ce que les éléments déflecteurs sont constitués de tiges déplaçables axialement (24), qui agissent sur le câble de fibres par des extrémités arrondies (25).

14. Dispositif selon l'une des revendications 11 à 13 caractérisé en ce que le dispositif déflecteur (20) pour le contrôle de la largeur du câble de fibres (2) présente au moins une tige de déviation courbe (26), dont l'axe longitudinal est perpendiculaire au sens de relèvement du câble et essentiellement parallèle au plan du câble, et il est pivotable autour de l'axe longitudinal du câble.

15. Dispositif selon l'une des revendications 11 à 14, caractérisé en ce que le dispositif déflecteur (22) pour le contrôle de la position du câble de fibres (2) présente dans le sens transversal au moins une tige, respectivement cylindre de déviation (28) droit, dont l'axe longitudinal est perpendiculaire au sens de relèvement du câble et parallèle au plan du câble et il est pivotable autour d'un axe qui passe parallèlement au sens d'élévation du câble.
